(19) Europäisches Patentamt
European Patent Office
Office européen des brevets

(11) Publication number: **0 153 418**
**A1**

(12) # EUROPEAN PATENT APPLICATION

published in accordance with Art. 158(3) EPC

(21) Application number: 84903200.8

(22) Date of filing: 27.08.84

Data of the international application taken as a basis:

(86) International application number: PCT/JP84/00412

(87) International publication number: WO85/01046 (14.03.85 85/07)

(51) Int. Cl.[4]: C 07 C 101/18
C 07 C 103/38, C 07 C 125/065
C 07 D 309/12, C 07 D 501/32
C 07 F 7/04

(30) Priority: 31.08.83 JP 160060/83
20.12.83 JP 241609/83

(43) Date of publication of application: 04.09.85 Bulletin 85/36

(84) Designated Contracting States: CH DE FR GB LI SE

(71) Applicant: Kyoto Pharmaceutical Industries, Ltd.
38, Nishinokyo Tsukinowa-cho Nakakyo-ku
Kyoto-shi Kyoto 604(JP)

(72) Inventor: NISHIZAWA, Susumu
1, Yamaden, Shimomisu Yokooji, Fushimi-ku
Kyoto-shi Kyoto 612(JP)

(72) Inventor: TAMAKI, Satoshi
1665-24, Kojima-cho
Moriyama-shi Shiga 524(JP)

(72) Inventor: KAWAMURA, Toshiyuki
14, Minami Ueda-cho Umezu, Ukyo-ku
Kyoto-shi Kyoto 615(JP)

(72) Inventor: KAKEYA, Nobuharu
10-16, Takadai 3-chome
Nagaokakyo-shi Kyoto 617(JP)

(72) Inventor: KITAO, Kazuhiko
12, Sandan Osa-cho Matsugasaki, Sakyo-ku
Kyoto-shi Kyoto 606(JP)

(74) Representative: von Kreisler, Alek, Dipl.-Chem et al,
Patentanwälte Dr.-Ing. Schönwald Dr.-Ing. Eishold; Dr. Fues Dipl.-Chem. von Kreisler; Dipl.Chem. Keller Dipl.-Ing. Selting; Dr. Werner Deichmannhaus am Hauptbahnhof
D-5000 Köln 1(DE)

(54) **MANDELIC ACID DERIVATIVES.**

(57) Mandelic acid derivatives represented by general formula (I), wherein $R_1$ represents a hydrogen atom or a hydroxy group, X represents a halogen atom or $OR_2$ (wherein $R_2$ represents a hydrogen atom, benzyl, benzhydryl, t-butyl, tetrahydropyranyl or silyl), and A represents an optionally protected amino acid residue, or salts thereof. These compounds are useful as intermediates for synthesizing cephalosporin derivatives, etc.

EP 0 153 418 A1

0153418

# SPECIFICATION

# Mandelic Acid Derivatives

## TECHNICAL FIELD

This invention relates to novel mandelic acid derivatives of the general formula

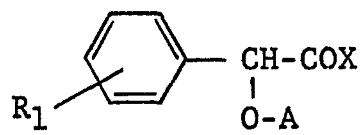

(I)

wherein $R_1$ is a hydrogen atom or a hydroxyl group, X is a halogen atom or the group $OR_2$ (in which $R_2$ is a hydrogen atom or a benzyl, benzhydryl, tert-butyl, tetrahydropyranyl or silyl group) and A is an amino acid residue which may optionally be protected on the amino group thereof, and salts thereof, said mandelic acid derivatives and salts thereof being useful as intermediates in the synthesis of cephalosporins, penicillins, etc.

## BACKGROUND ART

The present inventors previously developed cephalosporin derivatives of the general formula

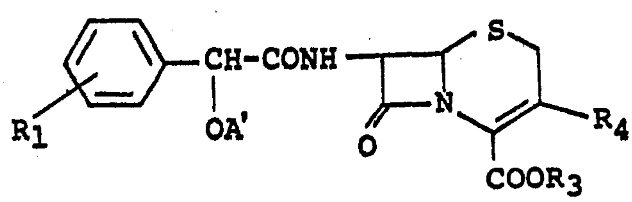

(A)

wherein A' is an α-, β- or γ-amino acid residue (bonded through an ester bonding) which may optionally be substituted by a lower alkyl group on the amino group thereof, $R_3$ is an 1-alkanoyloxyalkyl, 1-alkoxycarbonyloxyalkyl,

phthalidyl or 5-methyl-1,3-dioxolen-2-on-4-ylmethyl group, $R_4$ is a carbamoyloxymethyl group, which may optionally be substituted by a lower alkyl group or groups, or a heterocycle-thiomethyl group, which may optionally be substituted by an appropriate substituent or substituents, and $R_1$ is as defined above, and nontoxic salts thereof (cf. PCT/JP83/00437). Said cephalosporin derivatives (A) and salts thereof are excellent in absorbability through the digestive tract and, after absorption, are rapidly converted to the unesterified forms of the general formula

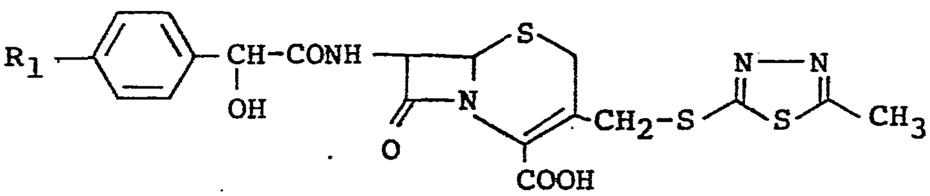 (B)

wherein $R_1$ is as defined above, which correspond to the cephalosporin derivatives (A), as a result of hydrolysis at the carboxylic acid ester moiety in position 4 of the cephem ring structure and at the amino acid ester moiety in position α in the 7-position side chain by enzymes occurring in the living body. Thus, oral administration of the cephalosporin derivatives (A) can result in high blood levels of the unesterified forms which have excellent antimicrobial activity. Therefore, the cephalosporin derivatives (A) and salts thereof are very excellent products.

The present inventors conducted various investigations to bring baout improvements in the method of producing

said novel cephalosporin derivatives (A) and salts thereof and found that the above mandelic acid derivatives (I) and salts thereof are very useful as raw materials for the production of said cephalosporin derivatives (A) and salts thereof.

OBJECT OF INVENTION

An object of the invention is to provide novel intermediates in the production of the cephalopsorin derivatives (A).

DISCLOSURE OF INVENTION

The present invention consists in the mandelic acid derivatives (I) and salts thereof.

Referring to general formula (I), the amino acid residue represented by A means an acyl group derived from the carboxyl group of an amino acid and may be an α-, β- or γ-amino acid residue. Said amino acid residue may optionally be substituted by a lower alkyl group or groups containing 1 to 4 carbon atoms, such as methyl, ethyl, propyl, isopropyl or butyl. Such amino acid residue may occur as the D, L or DL form and may comprise two or more amino acids constituting a peptide. Such amino acid residue includes, among others, the following:

Neutral amino acid residues:

Aliphatic amino acid residues (glycyl, alanyl, valyl, leucyl, isoleucyl, etc.), hydroxyamino acid residues (seryl, threonyl, etc.), sulfur-containing amino acid residues (cysteinyl, cystinyl, methionyl, etc.), amidoamino acid residues (asparaginyl, glutaminyl, etc.) and

aromatic amino acid residues (phenylalanyl, tyrosyl, tryptophyl, etc.);

Acidic amino acid residues:

Aspartyl, glutamyl, etc.;

Basic amino acid residues:

Histidyl, lysyl, arginyl, etc.;

Imino acid residues:

Prolyl, hydroxyprolyl, etc.;

Other amino acid residues than α-amino acids:

β-Alanyl, γ-aminobutyryl, etc.:

N-substituted amino acid residues:

Sarcosyl, N,N-dimethylglycyl, etc.;

Peptide residues:

Glycylglycyl.

The protective group for said amino acid residue may be any amino group-protecting group in conventional use in ordinary peptide synthesis and is, for example, benzyloxycarbonyl, p-methoxybenzyloxycarbonyl, trityl, tert-butoxycarbonyl, 2,2,2-trichloroethoxycarbonyl, formyl or 1-methyl-2-ethoxycarboylvinyl.

The salt of mandelic acid derivative (I) may be any pharmacologically acceptable salt and includes, among others, inorganic acid salts such as hydrochloride, sulfate, phosphate and nitrate and organic acid salts such as oxalate, fumarate, maleate, citrate, tartrate, methanesulfonate and toluenesulfonate.

The mandelic acid derivatives (I) according to the

invention can be produced, for example, by the method given below in terms of reaction equations.

In general formula (I), the halogen atom represented by X is preferably a chlorine, bromine or iodine atom. Referring to $R_2$, the silyl group is preferably trimethylsilyl, tert-butyldimethylsilyl or triethylsilyl, for instance.

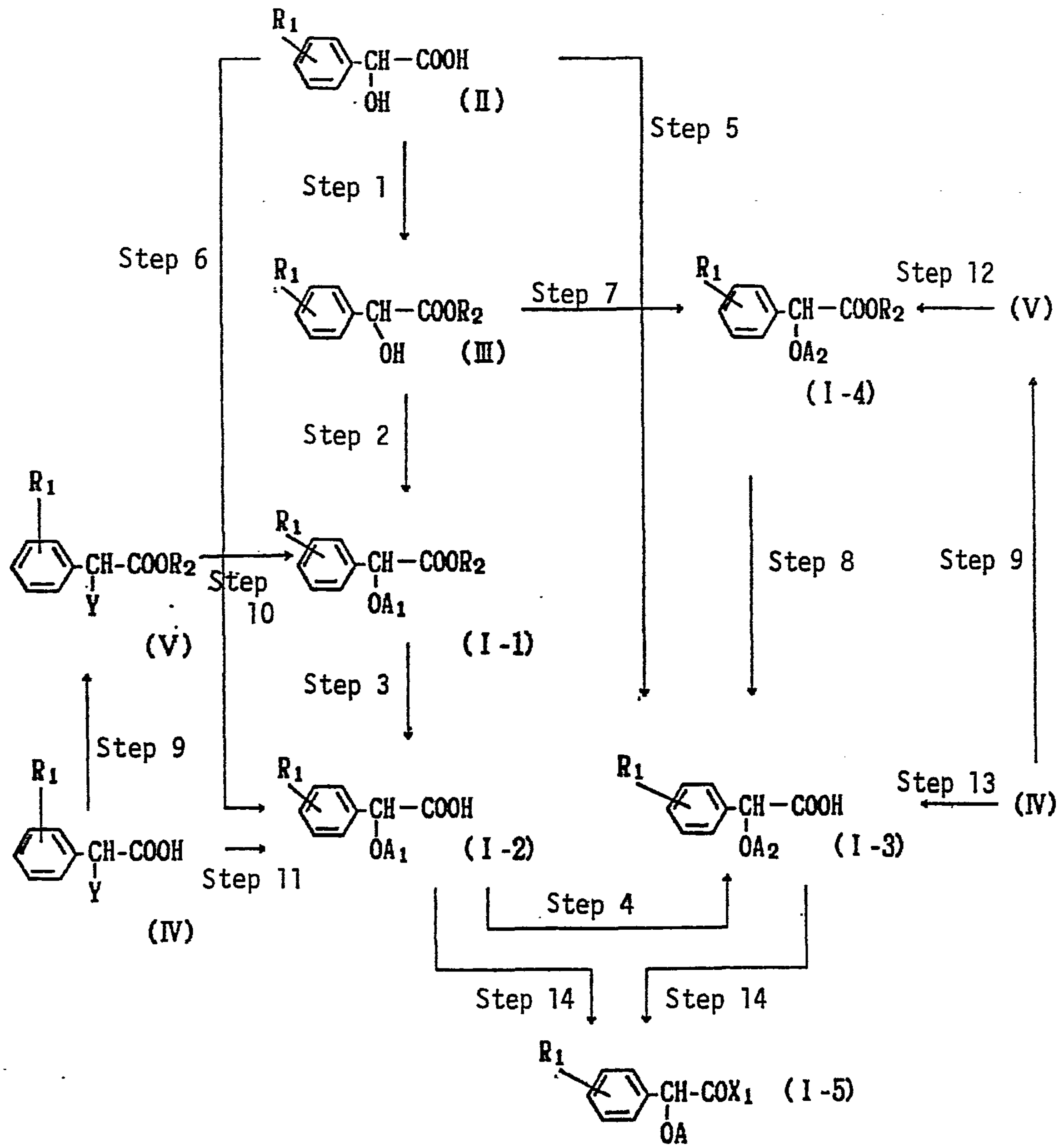


(In the above reaction equations, $R_2$ is benzyl, benzhydryl, tert-butyl, tetrahydropyranyl or silyl, $A_1$ is an amino acid residue having a protective group on the amino group thereof, $A_2$ has the same meaning as A', namely an amino acid residue having no protective group on the amino group thereof, Y is Cl, Br, I or tosylate, $X_1$ is a halogen atom, and A and $R_1$ are as defined above.)

Step I is a step of esterifying the compound (II) and is carried out by reacting the compound (II) with benzyl alcohol, benzyl halide, diphenyldiazomethane, tert-butanol or a silylating agent (e.g. trialkylsilyl halide such as trimethylsilyl chloride). The halide in said benzyl halide is preferably the chloride, bromide or iodide.

The compound (III) obtained in step I is substantially known and is described in Journal of Chemical Society, 2188 (1928).

Step 2 is a step of reacting the compound (III) with an amino acid or a reactive derivative thereof. The reactive derivative of amino acid to be used in this step is an ordinary reactive derivative at the carboxyl group and is, for example, an acid halide (e.g. acid chloride, acid bromide), a mixed acid anhydride (e.g. anhydride with monoethyl carbonate), an active ester (e.g. cyanomethyl ester, 4-nitrophenyl ester) or an active amide (e.g. amide with imidazole).

When the amino acid is used in the free form, it is preferable to use an appropriate condensation agent, such as an N,N'-disubstituted carbodiimide (e.g. N,N'-dicyclohexylcarbodiimide) or an azolide (e.g. N,N'-carbonyldiimidazole, N,N'-thionyldiimidazole). When such a condensation agent is used, the reaction presumably proceeds via a reactive derivative of the carboxylic acid.

This reaction is generally carried out in an inert

solvent. As the solvent, there may be mentioned more specifically, water, an organic solvent such as acetone, dioxane, acetonitrile, chloroform, benzene, methylene chloride, ethylene chloride, tetrahydrofuran, ethyl acetate, N,N-dimethylformamide or pyridine and a mixture of these.

The reaction is preferably carried out at room temperature or under cooling (-20°C to 0°C).

When, in compound (I-1), the protective group in the amino acid residue $A_1$ is benzyloxycarbonyl, $R_1$ is preferably tert-butyl or tetrahydropyranyl since said compound (I-1) is subsequently converted to compound (I-2).

Step 3 is a step of deprotecting compound (I-1). When $R_1$ is benzyl, the deprotection is preferably carried out by hydrogenation (e.g. catalytic reduction). The catalyst usable therein is, for example, palladium, platinum or Raney nickel. When $R_2$ is tert-butyl, tetrahydropyranyl or benzhydryl, the deprotection is preferably carried out by acid treatment, in which an inorganic acid such as hydrochloride or an organic acid such as trifluoroacetic acid or formic acid is used as the acid.

Step 4 is a step of deprotecting compound (I-2) to thereby convert the same to compound (I-3). The deprotection in this step may be conducted by a known method selected depending on the kind of protective group. For instance, tert-butoxycarbonyl and 1-methyl-2-ethoxycarbonyl may be eliminated by acid treatment, formyl by acid treatment, catalytic reduction or decomposition with

hydrazine, 2,2,2-trichloroethoxycarbonyl by treatment with methanol and zinc, and p-methoxybenzyloxycarbonyl by acid treatment or catalytic reduction.

Step 5 is a step of reacting compound (II) with a reactive derivative of an amino acid as prepared by modification of the carboxyl group thereof (e.g. N-carboxy-α-amino acid such as 4-methyloxazolidine-2,5-dione) to give compound (I-3).

The reaction in said step may be conducted by a *per se* known method.

Step 6 is a step of reacting compound (II) with a derivative of an amino-protected amino acid as prepared by modification of the carboxyl group thereof to give compound (I-2).

Step 7 is a step of reacting compound (III) with a reactive derivative of an amino acid with no protective group on its amino group as prepared by modification of the carboxyl group thereof to give compound (I-4).

The reaction in said step may be carried out by a *per se* known method.

Step 8 is a step of deprotecting compound (I-4) to give compound (I-3) and the deprotection may be conducted in the same manner as in step 3.

Step 9 is a step of protecting the carboxyl group of compound (IV) and is carried out by reacting compound (IV) with benzyl alcohol, benzyl halide, diphenyldiazomethane, tertiary butanol, dihydropyran or silylating

agent (e.g. trialkylsilyl halide such as trimethylsilyl chloride). The halide in said benzyl halide is preferably the chloride, bromide or iodide

Step 10 is a step of reacting compound (V) with a reactive derivative of an amino-protected amino acid. The reactive derivative of amino acid to be used in this step is, for example, the lithium, sodium, potassium or cesium salt or an organic amine salt (e.g. triethylamine salt, pyridine salt).

The reaction of this step is generally carried out in an inert solvent. Examples of the solvent are water, organic solvents such as acetpne, dioxane, acetonitrile, chloroform, benzene, methylene chloride, ethylene chloride, tetrahydrofuran, ethyl acetate, N,N-dimethylformamide and pyridine, and mixed solvents composed of these. This reaction is preferably carried out at about 50°C or below or with cooling (0° to 5°C).

Step 11 is a step of reacting compound (IV) with a reactive derivative of an amino-protected amino acid. The reaction of this step is carried out in substantially the same manner as in step 10.

Step 12 is a step of reacting compound (V) with an amino acid having no protective group on its amino group and is carried out in almost the same manner as step 10.

Step 13 is a step of reacting compound (IV) with an amino acid having no protective group on its amino group

and is carried out in the same manner as step 11.

Step 14 is a step of producing compound (I-5) by halogenating compound (I-2) or (I-3) with a per se known halogenating agent (e.g. phosphorus pentachloride, phosphorus oxychloride, thionyl chloride, phosgene; preferably phosphorus pentachloride). The halogenation reaction is generally carried out in an inert solvent (e.g. methylene chloride, dioxane, acetonitrile, chloroform, N,N-dimethylformamide, pyridine, mixture of these), at a reaction temperature of from about room temperature down to -20°C to 0°C (with cooling), and preferably with stirring.

The mandelic acid derivatives (I) are useful as acylating agents. For example, acylation of 7-aminocephalosporanic acid, esters thereof, 6-aminopenicillanic acid, esters thereof therewith can give cephalosporins and penicillins. More particularly, they are used as raw materials for the production of oral cephalosporins disclosed in PCT/JP83/00437.

The following examples illustrate the invention in more detail. They are, however, by no means limitative of the present invention.

In the description which follows, BOC means a tert-butoxycarbonyl group and ϕ a phenyl group.

Example 1

Synthesis of D-O-(BOC-L-alanyl)mandelic acid benzyl ester:

(Method 1)

In 25 ml of methylene chloride, there are dissolved 2.42 g of D-mandelic acid benzyl ester, 62 mg of 4-dimethylaminopyridine and 1.89 g of BOC-L-alanine. To the solution is added 11 ml of 1 M dicyclohexylcarbodiimide in methylene chloride at -5°C to 0°C, followed by stirring at the same temperature for 30 minutes. Thereafter, the resulting dicyclohexylurea is filtered off, and the filtrate is washed in sequence with saturated aqueous sodium bicarbonate, 10% aqueous citric acid and saturated aqueous sodium chloride. After drying over anhydrous sodium sulfate, the solvent is distilled off under reduced pressure. Ethyl acetate is added to the residue and the resulting precipitate, dicyclohexylurea, is again filtered off. The filtrate is evaporated to dryness under reduced pressure. Addition of n-hexane to the residue gives 2.75 g (67% yield) of the title compound.

IR (nujol, $cm^{-1}$) ; 3370, 1740, 1690

NMR ($CDCl_3$, δ value)

1.41 (s, 9H, $-C(CH_3)_3$)

1.42 (d, 3H, J=7Hz, $CH_3$-CH-)

4.48 (m, 1H, $CH_3$-CH-)

4.8 ∿ 5.2 (br, 1H, -NH-)

5.16 (s, 2H, $-CH_2$-ϕ)

6.01 (s, 1H, ϕ-CH-)

7.24, 7.38 (2 x s, 10H, -ϕ x 2)

(Method 2)

In 25 ml of tetrahydrofuran, there are dissolved 242 mg of D-mandelic acid benzyl ester and 286 mg of BOC-L-alanine N-hydroxysuccinimide ester, and the solution is heated under reflux for 4 hours. Thereafter, ethyl acetate is added, and the resulting mixture is washed with saturated aqueous sodium bicarbonate and saturated aqueous sodium chloride. After drying over anhydrous sodium sulfate, the solvent is distilled off under reduced pressure. The residue is purified using 10 g of silica gel (eluent: benzene-ethyl acetate 10:1) and crystallized from n-hexane to give 200 mg of the title compound.

(method 3)

BOC-L-Alanine (189 mg) is dissolved in 2 ml of tetrahydrofuran, followed by addition of 1 ml of 1 M triethylamine in tetrahydrofuran and cooling to -15°C. After addition of 1 ml of 1 M ethyl chloroformate in tetrahydrofuran, the resulting mixture is stirred for 5 minutes. Then, 121 mg of 4-dimethylaminopyridine and 243 mg of D-mandelic acid benzyl ester are added, and the mixture is stirred at -15°C to 0°C for 2 hours and then at room temperature for 3 hours. Thereafter, ethyl acetate and water are added, and the ethyl acetate layer is washed with saturated aqueous sodium bicarbonate and saturated aqueous sodium chloride. After drying over sodium sulfate, the solvent is distilled off under reduced pressure. The residue is purified using 10 g of silica

gel (eluent: benzene-ethyl acetate 10:1) and crystallized from n-hexane to give 120 mg of the title compound.

Example 2

Synthesis of D-O-(BOC-L-alanyl)mandelic acid:

In 100 ml of ethyl acetate is dissolved 12 g of the benzyl ester obtained in Example 1. After addition of 1 g of 5% palladium-on-carbon, the ester is catalytically reduced. After the reaction, the catalyst is filtered off, and the filtrate is evaporated under reduced pressure. Crystallization by adding n-hexane to the residue gives 8 g (85% yield) of the title compound.

IR (nujol, $cm^{-1}$) ; 3500, 3425, 1725, 1690

NMR ($CDCl_3$, δ value)

1.38 (s, 9H, $-C(CH_3)_3$)

1.40 (d, 3H, J=7Hz, $\underline{CH_3}$-CH-)

4.40 (m, 1H, $CH_3$-$\underline{CH}$-)

6.00 (s, 1H, ϕ-$\underline{CH}$-)

7.2 ∿ 7.5 (m, 6H, ϕ, -COOH)

9.27 (br. s, 1H, -NH-)

Example 3

Synthesis of D-O-(L-alanyl)mandelic acid benzyl ester:

Powder-form L-alanine (3 g) is added to 200 ml of anhydrous tetrahydrofuran, followed by addition, under stirring, of a solution of 7 g of phosgene in 50 ml of

anhydrous tetrahydrofuran. The mixture is stirred at 40°C until it becomes a uniform solution. The solvent is then distilled off under reduced pressure, 100 ml of dried ether, 7.4 g of D-mandelic acid benzyl ester and 50 mg of 4-dimethylaminopyridine are added, and the mixture is stirred overnight. The ether is distilled off, ethyl acetate is added, and the mixture is washed with aqueous sodium bicarbonate and aqueous sodium chloride. After drying over anhydrous sodium sulfate, the solvent is distilled off. There is obtained 2.7 mg of the title compound.

NMR ($CDCl_3$, δ value)

1.18 (d, 3H, J=7Hz, $-\overset{|}{C}HC\underline{H_3}$)

4.3 (m, 1H, $CH_3-\overset{|}{C}\underline{H}-$)

5.08 (s, 2H, $-OCH_2-$)

6.09 (s, 1H, -CHO-)

7.0 ∿ 7.45 (m, 10H, φ)

8 ∿ 10 (br., 2H, $-NH_2$)

Example 4

Synthesis of D-O-(L-alanyl)mandelic acid hydrochloride:

In 5 ml of 99% formic acid is dissolved 2.4 g of the compound obtained in Example 2. With ice cooling, 15 ml of 4 N hydrogen chloride in isopropyl alcohol is added, and the mixture is stirred for 10 minutes. Thereafter, the reaction mixture is poured into ether, and the

resulting crystals are washed with eth r and dried to give 1.2 g (62% yield) of the title compound

IR (KBr, $cm^{-1}$) ; 1755, 1745

NMR ($DMSO-d_6$, δ value)

1.49 (d, 3H, J=7Hz, $C\underline{H_3}$-CH-)

4.20 (m, 1H, $CH_3$-C$\underline{H}$-)

6.01 (s, 1H, ϕ-C$\underline{H}$-)

7.2 ∿ 7.75 (m, 5H, -ϕ)

8.00 ∿ 9.80 (br, 4H, $-NH_3^{\oplus}$, $-CO_2H$)

$[\alpha]_D^{31}$ = -130.63° (C=3%, methanol)

Example 5

Synthesis of D-O-(N-benzyloxycarbonyl-L-alanyl)-mandelic acid benzhydryl ester:

In 15 ml of methylene chloride, there are dissolved 1.19 g of D-(-)-mandelic acid benzhydryl ester, 0.83 g of N-benzyloxycarbonyl-L-alanine and 20 mg of 4-dimethylaminopyridine. At -5°C to 0°C, 3.7 ml of 1 M dicyclohexylcarbodiimide in methylene chloride is added, and the mixture is stirred at the same temperature for 30 minutes. Thereafter, the reaction mixture is treated in the same manner as in Method 1 in Example 1 to give 1.8 g of the title compound as an oil.

IR (nujol, $cm^{-1}$) ; 1740, 1690

NMR ($CDCl_3$, δ value)

1.40 (d, 3H, J=7Hz, $C\underline{H_3}$-CH-)

4.57 (m, 1H, $CH_3$-CH-)

5.08 (s, 2H, $-CH_2-$)

5.30 (br, 1H, -NHCH-)

6.09 (s, 1H, ϕ-CH-)

6.84 (s, 1H, $-CO_2CH-$)

7 ∿ 7.4 (m, 20H, phenyl)

Example 6

Synthesis of D-O-(formyl-L-alanyl)mandelic acid tert-butyl ester:

To 100 ml of methylene chloride, there are added 12.6 g of formyl-L-alanine, 24.2 g of D-mandelic acid tert-butyl ester and 50 mg of 4-dimethylaminopyridine, followed by dropwise addition of 100 ml of 1 N dicyclohexylcarbodiimide in methylene chloride at 0°C. The resulting mixture is stirred at the same temperature for 30 minutes and then at room temperature for 1 hour, the precipitate is filtered off, and the solvent is removed under reduced pressure. Ethyl acetate is added to the concentrate, the resulting precipitate urea compound is filtered off, and the ethyl acetate is distilled off under reduced pressure. Thus is obtained 34 g of the title compound (yield: 100%).

NMR ($CDCl_3$, δ value)

1.4 (s, 9H, $-(CH_3)_3$)

1.45 (d, 3H, J=7Hz, $-CHCH_3$)

4.87 (q, 1H, J=7Hz, -CHCH$_3$)

5.99 (s, 1H, -CHO-)

6.2 (br, 1H, -NH-)

7.36 (s, 5H, ϕ)

8.12 (s, 1H, -CHO)

Example 7

Synthesis of D-O-(N-benzyloxycarbonyl-L-alanyl)-mandelic acid:

In 5 ml of methylene chloride is dissolved 524 mg of the compound obtained in Example 5. With ice cooling, 3.8 ml of trifluoroacetic acid is added, and the mixture is stirred for 1 hour. Thereafter, the reaction mixture is evaporated to dryness under reduced pressure, ethyl acetate is added to the residue, and the mixture is extracted with saturated aqueous sodium bicarbonate. The aqueous layer obtained is made acid with 10% aqueous citric acid and extracted with ethyl acetate. The ethyl acetate layer is washed with saturated sodium chloride and dried over anhydrous sodium sulfate, and the solvent is then distilled off under reduced pressure. Thus is obtained 260 mg (73% yield) of the title compound.

IR (nujol, $cm^{-1}$) ; 1730, 1680

NMR ($CDCl_3$, δ value)

1.40 (d, 3H, J=7Hz, CH$_3$-CH-)

4.50 (m, 1H, CH$_3$-CH-)

5.06 (s, 2H, $-CH_2-$)

5.3 ∿ 5.7 (br, 1H, -NH-)

5.98 (s, 1H, $\phi$-CH-)

7.29, 7.37 (2 x s, 10H, $-\phi$ x 2)

8.30 (br, s, 1H, $-CO_2H$)

Example 8

Synthesis of D-O-(BOC-L-alanyl)mandelic acid:

BOC-L-alanine (2 g) is dissolved in 30 ml of anhydrous tetrahydrofuran, followed by addition of 1.1 g of triethylamine and further by dropwise addition of a solution of 1.14 g of ethyl chloroformate in 10 ml of anhydrous tetrahydrofuran at -20°C. The resulting mixture is stirred at the same temperature for 30 minutes. Thereto are added 1.61 g of D-mandelic acid and 50 mg of 4-dimethylaminopyridine, and the mixture is stirred at room temperature for 2 hours. The solvent is then distilled off under reduced pressure, ethyl acetate is added to dissolve the residue, the solution is washed with aqueous sodium chloride and dried over anhydrous sodium sulfate, the solvent is distilled off under reduced pressure, and hexane is added. Upon standing, there is obtained 830 mg of the title compound (the same compound as that of Example 2). (Yield: 24%).

Example 9

In 40 ml of ethyl acetate is dissolved 3.6 g of the compound obtained in Example 5. After addition of 180 mg

of palladium oxide, said compound is catalytically reduced. After the reaction, the catalyst is filtered off, hydrogen chloride is blown into the filtrate, the resulting crystalline precipitate is collevted by filtration. Thus is obtained 2.2 g (85% yield) of the same compound as that of Example 4.

Example 10

Powder-form L-alanine (4.5 g) was added to 200 ml of dried tetrahydrofuran, followed by dropwise addition of a solution of 6 g of phosgene in 50 ml of tetrahydrofuran with vigorous stirring. The resulting mixture was stirred at 40°C until it became homogeneous. The solvent was then distilled off under reduced pressure. D-Mandelic acid (7.6 g) and 200 ml of anhydrous ether were added, and the mixture was stirred overnight. The crystalline precipitate was collected by filtration and washed with ether and tetrahydrofuran to give 700 mg of the same substance as that of Example 4. (Yield: 5.4%)

Examples 11-13

Mandelic acid derivatives (I) shown in Table 1 were obtained by following the procedures of Examples 1, 2 and 4 or of Examples 5, 7 and 9.

0153418

Table 1

| Example | Structural formula | Physical characteristics |
|---|---|---|
| 11 | 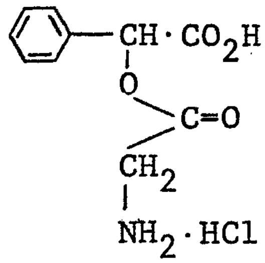<br>D-O-Glycylmandelic acid hydrochloride | IR (KBr, $cm^{-1}$) 1755, 1720<br>NMR ($DMSO-d_6$, δ value)<br>3.93 (s, 2H, $>CH_2$)<br>6.01 (s, 1H, φ-CH)<br>7.15 ∿ 7.70 (m, 5H, φ)<br>7.80 ∿ 9.60 (br, 4H, $-NH_3^{\oplus}$, $-CO_2H$)<br>$[\alpha]_D^{31}$ = -128.24°<br>(C = 3%, methanol) |
| 12 | 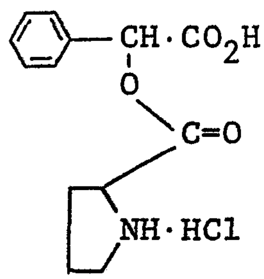<br>D-O-(L-Prolyl)-mandelic acid hydrochloride | IR (KBr, $cm^{-1}$) 1750, 1730<br>NMR ($DMSO-d_6$, δ value)<br>1.60 ∿ 2.65 (m, 4H, $>CH_2$ at positions 3 & 4 of proline)<br>3.00 ∿ 3.55 (m, 2H, $>CH_2$ at position 2 of proline)<br>4.30 ∿ 4.80 (m, 1H, -CH at position 5 of proline)<br>6.04 (s, 1H, φ-CH)<br>7.20 ∿ 7.25 (m, 5H, -φ)<br>8.70 ∿ 10.60 (br, 4H, $-NH_3^{\oplus}$ $-CO_2H$)<br>$[\alpha]_D^{31}$ = -140.94°<br>(C = 3%, methanol) |
| 13 | 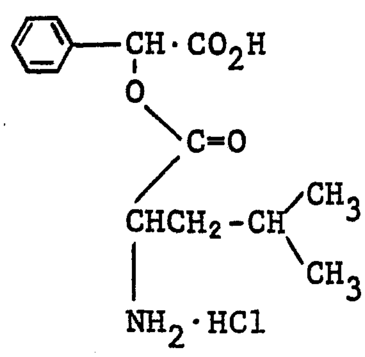<br>D-O-(L-Leucyl)-mandelic acid hydrochloride | IR (KBr, $cm^{-1}$) 1750, 1740<br>NMR ($DMSO-d_6$, δ value)<br>0.88 (d, 6H, J=5Hz, $-CH-(CH_3)_2$)<br>1.30 ∿ 2.25 (m, 3H, $-CH_2CH-$)<br>3.75 ∿ 4.25 (m, 1H, -CO-CH-)<br>5.99 (s, 1H, φ-CH)<br>7.10 ∿ 7.70 (m, 5H, -φ)<br>7.40 ∿ 9.30 (br, 4H, $-NH_3^{\oplus}$, $-CO_2H$) |

Example 14

Production of O-L-alanyl-D-mandelic acid chloride hydrochloride:

In 140 ml of methylene chloride, there was suspended 7 g of D-O-(L-alanyl)mandelic acid hydrochloride obtained in Example 4, followed by addition of 140 ml of 8% phosphorus pentachloride in methylene chloride with cooling. The mixture was stirred overnight and then filtered to give 7.5 g (94% yield) of the title compound.

$[\alpha]_D^{28}$ = -56.28°(c = 1, dioxane)

IR (nujol, $cm^{-1}$): 3020, 1790, 1750

Example 15

Production of D-O-(L-alanyl)mandelic acid hydrochloride:

α-Bromophenylacetic acid (2.1 g) was dissolved in 5 ml of dried dimethylformamide. N-Trimethylsilylacetamide (2.6 g) was added, followed by addition of 2.4 g of potassium salt of N-(1-methyl-2-ethoxycarbonylvinyl)-L-alanine. The mixture was stirred at 30°C for 16 hours. To the reaction mixture was added 1.1 ml of 10 N ethanolic hydrochloride. The mixture was stirred for 30 minutes and poured into ether. The resulting precipitate was purified using an Amberlite XAD-II column (elution with water) and the eluate fraction was lyophilized. Recrystallization from methanol-ethyl acetate and then from methanol-ether gave 330 mg of the title compound.

Example 16

Synthesis of 4-hydroxy-D-O-(BOC-L-alanyl)mandelic acid:

1) 4-Hydroxy-D-mandelic acid (6.2 g) is dissolved in 50 ml of acetonitrile. Diphenyldiazomethane (7.9 g) was added at 0°C and the mixture is stirred at the same temperature for 1 hour. The precipitate is collected by filtration and washed with isopropyl ether. The thus-obtained benzhydryl ester (9.7 g) is dissolved in 40 ml of dried tetrahydrofuran, and 19.8 ml of 30% carbobenzoxy chloride in toluene. Further, 4.25 ml of triethylamine is added dropwise gradually, and the mixture is stirred for 30 minutes. The solvent is distilled off under reduced pressure, ethyl acetate is added to the residue, the mixture is washed with aqueous sodium carbonate and aqueous sodium chloride and dried over anhydrous sodium sulfate, the solvent is distilled off under reduced pressure, and the residue is crystallized from petroleum ether and ethyl acetate. In this manner, 12.3 g of 4-benzyloxycarbonyloxy-D-mandelic acid benzhydryl ester. (Yield: 71%)

IR (nujol, $cm^{-1}$) : 3450, 1760, 1740

NMR ($DMSO-d_6$, δ value)

5.28 (s, 2H, $CH_2$)

5.34 (d, 1H, J=6Hz, $-\overset{|}{C}\underline{H}-OH$)

6.22 (d, 1H, J=6Hz, -OH)

6.79 (s, 1H, $-C\underline{H}\phi_2$)

6.9 ∿ 7.7 (m, 19H, phenyl)

2) BOC-L-alanine (5.34 g) is dissolved in 300 ml of methylene chloride and the solution is cooled to 0°C. 1 M Dicyclohexylcarbodiimide in methylene chloride (28.3 ml) is added and, thereafter, 11.5 g of the compound obtained in 1) and 150 mg of 4-dimethylaminopyridine are added, and the mixture is stirred at the same temperature for 1 hour. The precipitate is filtered off and the solvent is distilled off under reduced pressure. Ethyl acetate is added to the residue, the solution is washed with 10% aqueous citric acid and aqueous sodium chloride and dried over anhydrous sodium sulfate, and the solvent is distilled off under reduced pressure, followed by recrystallization from petroleum ether and ethyl acetate. In this manner, there was obtained 13.8 g of 4-benzyloxycarbonyloxy-D-O-(BOC-L-alanyl)mandelic acid benzhydryl ester. (Yield: 88%).

IR (nujol, $cm^{-1}$) : 3380, 1765, 1750, 1700

NMR (DMSO-$d_6$, δ value)

1.27 (d, 3H, J=7Hz, $\underline{CH_3}\overset{|}{C}H$)

1.32 (s, 9H, $-C(\underline{CH_3})_3$)

4.18 (m, 1H, $-\overset{|}{C}\underline{H}NH$)

5.29 (s, 2H, $CH_2$)

6.22 (s, 1H, $\phi\overset{|}{C}\underline{H}-$)

6.82 (s, 1H, $-C\underline{H}\phi_2$)

7.0 ∿ 7.7 (m, 19H, phenyl)

3) In 150 ml of tetrahydrofuran containing 1% of water, there is dissolved 13 g of the product of 2), and hydrogenation is performed with 1 g of palladium oxide as the catalyst. The catalyst is filtered off, the solvent is distilled off under reduced pressure, and the solid thus obtained is washed with petroleum ether. In this way, 6 g of the title compound was obtained. (Yield: 87%)

IR (nujol, $cm^{-1}$) : 3450, 3340, 1745, 1660

NMR ($DMSO-d_6$, δ value)

1.28 (d, 3H, J=7Hz, $\underline{CH_3}\overset{|}{C}H$)

1.34 (s, 9H, $C(CH_3)_3$)

4.1 (m, 1H, $-\overset{|}{C}\underline{H}NH$)

5.7 (s, 1H, $\phi\overset{|}{C}\underline{H}-$)

6.95, 7.24 (d, d, 4H, J=8Hz, phenyl)

7.2, 9.6, 12.8 (3br, 3H, 2 x OH, NH)

$[\alpha]_D^{28}$ -146.6° (c = 1. methanol)

Reference Example 1

Production of (5-methyl-1,3-dioxol-2-on-4-yl)methyl 7-[D-O-(L-alanyl)mandelamido]-3-[(5-methyl-1,3,4-thiadiazol-2-yl)thiomethyl]-3-cephem-4-carboxylate hydrochloride:

In 150 ml of methylene chloride, there are suspended 5 g of O-L-alanyl-D-mandelic acid chloride hydrochloride and 3.05 g of (5-methyl-1,3-dioxol-2-on-4-yl)methyl 7-amino-3-[(5-methyl-1,3,4-thiadiazol-2-yl)thiomethyl]-3-cephem-4-carboxylate. The suspension is cooled to -10°C, a mixture of 2.23 g of triethylamine and 10 ml of methylene chloride is added dropwise over 10 minutes and, after the addition, the mixture is stirred for 20 minutes, then washed three times with saturated aqueous sodium chloride and dried over anhydrous sodium sulfate. The solvent is distilled off under reduced pressure, 100 ml of chloroform is added for dissolving the residue, 2 ml of 5 N hydrogen chloride in isopropyl alcohol to thereby cause crystallization and, after addition of 300 ml of ether, the crystals are collected by filtration and recrystallized from methanol and acetone or from ethanol and acetone to give 5 g of the title compound.

IR (nujol, $cm^{-1}$) : 1815, 1780, 1690

NMR ($(CD_3)_2SO$, δ value)

1.48 (d, 3H, J=7Hz, $-\overset{|}{C}H\underline{CH_3}$)

2.18 (s, 3H, dioxole-$CH_3$)

2.66 (s, 3H, thiadiazole-$CH_3$)

3.62 (br. s, 2H, 2-position-$H_2$)

4.20 (m, 1H, $-\overset{|}{C}\underline{H}CH_3$)

4.07, 4.65 (d, d, 2H, J=14Hz, 3-position-$CH_2S$-)

5.05 (d, 1H, J=5Hz, 6-position-H)

5.15 (s, 2H, dioxole-$CH_2$-)

5.72 (d x d, 1H, J=5 and 9Hz, 7-position-H)

6.13 (s, 1H, -CHCONH-)

7.63 (m, 5H, phenyl)

8.73 (br, 3H, $-NH_3$)

9.46 (d, 1H, J=9Hz, -CONH-)

CLAIMS

1. A mandelic acid derivative or a salt thereof, said mandelic acid derivative having the general formula

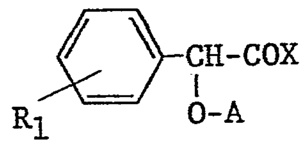

(I)

wherein $R_1$ is a hydrogen atom or a hydroxyl group, X is a halogen atom or the group $OR_2$ (in which $R_2$ is a hydrogen atom or a benzyl, benzhydryl, tert-butyl, tetrahydropyranyl or silyl group) and A is an amino acid residue which may optionally be protected on the amino group thereof.

2. A mandelic acid derivative or salt thereof as set forth in Claim 1, wherein the amino acid residue is an α-amino acid residue.

3. A mandelic acid derivative (I) as set forth in Claim 1, said mandelic acid derivative being D-O-(BOC-L-alanyl)mandelic acid benzyl ester.

4. A mandelic acid derivative (I) as set forth in Claim 1, said mandelic acid derivative being D-O-(N-benzyloxycarbonyl-L-alanyl)mandelic acid benzhydryl ester.

5. A mandelic acid derivative (I) as set forth in Claim 1, said mandelic acid derivative being D-O-(formyl-L-alanyl)mandelic acid tert-butyl ester.

6. A mandelic acid derivative (I) as set forth in Claim 1, said mandelic acid derivative being D-O-(BOC-L-alanyl)mandelic acid.

7. A mandelic acid derivative (I) as set forth in Claim 1, said mandelic acid derivative being D-O-(N-benzyloxycarbonyl-L-alanyl)mandelic acid.

8. A mandelic acid derivative (I) as set forth in Claim 1 or a salt thereof, said mandelic acid derivative being D-O-(L-alanyl)mandelic acid benzyl ester.

9. A mandelic acid derivative (I) as set forth in Claim 1 or a salt thereof, said mandelic acid derivative being D-O-(L-alanyl)mandelic acid.

10. A mandelic acid derivative (I) as set forth in Claim 1 or a salt thereof, said mandelic acid derivative being D-O-glycyl-mandelic acid.

11. A mandelic acid derivative (I) as set forth in Claim 1 or a salt thereof, said mandelic acid derivative being D-O-(L-prolyl)mandelic acid.

12. A mandelic acid derivative (I) as set forth in Claim 1 or a salt thereof, said mandelic acid derivative being D-O-(L-leucyl)mandelic acid.

13. A mandelic acid derivative (I) as set forth in Claim 1 or a salt thereof, said mandelic acid derivative being O-L-alanyl-D-mandelic acid.

14. A mandelic acid derivative (I) as set forth in Claim 1, said mandelic acid derivative being 4-hydroxy-D-O-(BOC-L-alanyl)mandelic acid.

# INTERNATIONAL SEARCH REPORT



International Application No. PCT/JP84/00412

**I. CLASSIFICATION OF SUBJECT MATTER** (if several classification symbols apply, indicate all) [3]

According to International Patent Classification (IPC) or to both National Classification and IPC

Int.Cl[3] C07C101/18, C07C103/38, C07C125/065
C07D309/12, C07D501/32, C07F7/04

**II. FIELDS SEARCHED**

Minimum Documentation Searched [4]

| Classification System | Classification Symbols |
|---|---|
| IPC | C07C101/18, C07C103/38, C07C125/065<br>C07D309/12, C07D501/32, C07F7/04 |

Documentation Searched other than Minimum Documentation
to the Extent that such Documents are Included in the Fields Searched [5]

**III. DOCUMENTS CONSIDERED TO BE RELEVANT** [14]

| Category* | Citation of Document, [16] with indication, where appropriate, of the relevant passages [17] | Relevant to Claim No. [18] |
|---|---|---|
| A | JP, B2, 54-14113 (Fujisawa Pharmaceutical Co., Ltd.), 05 June 1979 (05. 06. 74) | 1 - 14 |

* Special categories of cited documents: [15]
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance: the claimed invention cannot be considered novel or cannot be considered to involve an inventive step
"Y" document of particular relevance; the claimed invention cannot be considered to involve an inventive step when the document is combined with one or more other such documents, such combination being obvious to a person skilled in the art
"&" document member of the same patent family

**IV. CERTIFICATION**

| Date of the Actual Completion of the International Search [2] | Date of Mailing of this International Search Report [2] |
|---|---|
| November 5, 1984 (05. 11. 84) | November 19, 1984 (19. 11. 84) |
| International Searching Authority [1] | Signature of Authorized Officer [20] |
| Japanese Patent Office | |